# EUROPÄISCHE PATENTSCHRIFT

(11) **EP 0 912 540 B1**
(45) Veröffentlichungstag und Bekanntmachung des Hinweises auf die Patenterteilung: **11.12.2002**
(21) Anmeldenummer: 97928217.5
(22) Anmeldetag: 17.06.1997
(51) Int. Cl.: C07D 307/88

(54) **VERFAHREN ZUR HERSTELLUNG VON PHTHALIDEN**
PROCESS FOR PREPARING PHTHALIDES
PROCEDE DE PREPARATION DE PHTHALIDES

(30) Priorität: 03.07.1996 DE 19626659
(43) Veröffentlichungstag der Anmeldung: 06.05.1999
(73) Patentinhaber: BASF AKTIENGESELLSCHAFT, 67056 Ludwigshafen (DE)
(72) Erfinder: MASSONNE, Klemens, D-67368 Westheim (DE); BECKER, Rainer, D-67098 Bad Dürkheim (DE); REIF, Wolfgang, D-67227 Frankenthal (DE); NEUHAUSER, Horst, D-67373 Dudenhofen (DE); GIESELER, Andreas, D-67098 Bad Dürkheim (DE); MUNDINGER, Klaus, D-67117 Limburgerhof (DE)
(86) Internationale Anmeldenummer: EP9703137
(87) Internationale Veröffentlichungsnummer: WO98001437

(56) Entgegenhaltungen:
- EP-A- 0 542 037
- DE-A- 2 803 319
- DE-A- 3 201 300

## Beschreibung

Die vorliegende Erfindung betrifft ein Verfahren zur Herstellung von Phthaliden durch katalytische Hydrierung von Phthalsäureanhydriden in einem Lösungsmittel, bei dem das Phthalid selbst als Lösungsmittel verwendet wird.

Aus DE-C-28 03 319 ist ein Verfahren zur Herstellung von Phthalid durch katalytische Hydrierung von Phthalsäureanhydrid in der Gasphase bekannt, was jedoch einen erheblichen Aufwand bei der Produktisolierung durch mehrstufige Kondensation und der damit verbundenen Abgaswäsche erfordert.

Es ist weiter bekannt, die katalytische Hydrierung von Phthalsäureanhydrid zu Phthalid in inerten Lösungsmitteln wie Tetrahydrofuran (EP-A-0 542 037), Benzoesäureester gegebenenfalls im Gemisch mit Alkoholen (EP-B-0 089 417), Ester niederer einbasiger Alkohole mit niederen Fettsäuren (US-A-2 079 325), niedere einbasige aliphatische Alkohole (US-A 2 114 696) durchzuführen.

Die bekannten Verfahren zu katalytischen Hydrierung von Phthalsäureanhydriden zu Phthalid in einem Lösungsmittel haben den Nachteil, daß als Reaktionsprodukte Gemische von Phthalid und Lösungsmittel erhalten werden, die durch aufwendige Trennoperationen wie Destillation und/oder Extraktion getrennt werden müssen, um die gewünschten Phthalide von den eingesetzten Lösungsmitteln zu befreien. Bei technischen Anwendungen müssen vor dem Hintergrund der aus ökologischen und ökonomischen Gründen notwendigen Wiederverwendung der Lösungsmittel im allgemeinen zusätzliche Verfahrensschritte zur Lösungsmittelaufarbeitung durchgeführt werden.

Der vorliegenden Erfindung lag daher die Aufgabe zugrunde, den zuvor genannten Nachteilen bei der Verwendung von Lösungsmitteln bei der katalytischen Hydrierung von Phthalsäureanhydriden abzuhelfen. Demgemäß wurde ein neues und verbessertes Verfahren zur Herstellung von Phthaliden der allgemeinen Formel I, 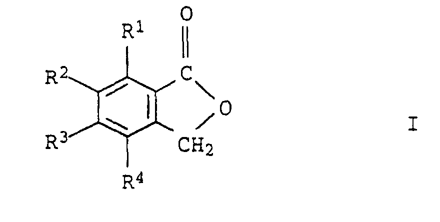 in der R¹, R², R³ und R⁴ unabhängig voneinander wasserstoff, C₁-C₄-Alkyl oder C₁-C₄-Alkoxy bedeuten, durch katalytische Hydrierung von Phthalsäureanhydriden der allgemeinen Formel II, 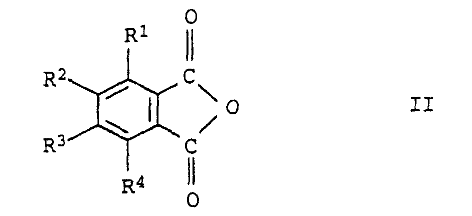 in der die Reste R¹, R², R³ und R⁴ die oben genannten Bedeutungen haben, in einem Lösungsmittel gefunden, welches dadurch gekennzeichnet ist, daß man das Phthalid I, das bei der katalytischen Hydrierung als Reaktionsprodukt entsteht, als Lösungsmittel verwendet.

Es war überraschend, daß das bei der katalytischen Hydrierung des Phthalsäureanhydrids entstehende Phthalid anstelle eines inerten organischen Lösungsmittels als Lösungsmittel verwendet werden kann, da bekannt ist, daß Phthalid weiterhydriert werden kann unter Bildung von störenden Nebenprodukten wie o-Tolylsäure und von im aromatischen Kern hydrierten Produkten, die besonders bei der Reinigung des Rohphthalids stören und deren Abtrennung sehr aufwendig ist.

Gegenüber der Verwendung der bekannten organischen Lösungsmittel hat das erfindungsgemäße Verfahren den Vorteil, daß die erhaltenen Reaktionsgemische im wesentlichen aus dem Phthalid-Produkt mit geringen Mengen an Nebenprodukten bestehen. Bei der Isolierung des Phthalids entfällt somit der Schritt der Lösungsmittelabtrennung und die für die Wiederverwendung des Lösungsmittels erforderlichen zusätzlichen Reinigungsschritte für das Lösungsmittel. Das neue Verfahren zeichnet sich somit gegenüber den bekannten Verfahren durch eine einfachere Handhabung und durch eine erhöhte Wirtschaftlichkeit aus.

Ein weiterer überraschender Vorteil der Verwendung von Phthalid als Lösungsmittel gegenüber der Verwendung von den aus dem Stand der Technik bekannten Lösungsmitteln, z.B. gegenüber der in EP-A-0 542 037 beschriebenen Verwendung von Butyrolacton als Lösungsmittel, besteht darin, daß die Hydrierung des Phthalsäureanhydrids zum Phthalid rascher abläuft. So ergibt sich beispielsweise beim Vergleich der Hydriergeschwindigkeiten in Butyrolacton und in Phthalid als Lösungsmittel, daß die Hydriergeschwindigkeit in Phthalid gegenüber der in Butyrolacton um etwa den Faktor 3 höher liegt.

Bei der Durchführung des erfindungsgemäßen Verfahrens kann das als Lösungsmittel zu verwendende Phthalid entweder als solches für die Reaktion dem eingesetzten Phthalsäureanhydrid zugesetzt werden oder man geht zunächst vom Phthalsäureanhydrid aus und nutzt das bei der Hydrierung des Phthalsäureanhydrids entstehende Phthalid als Reaktionsmedium. Beim Einsatz von hochschmelzenden Phthalsäureanhydriden kann es vorteilhaft sein, von Anfang an das Phthalid als Lösungsmittel zu verwenden, da die Gemische Phthalid/Phthalsäureanhydrid einen niedrigeren Schmelzpunkt als die reinen Verbindungen aufweisen. Zudem ist aus solchen Gemischen durch die Verdünnung des Phthalsäureanhydrids mit dem Phthalid die Reaktionswärme von Beginn an leichter abzuführen, so daß diese Fahrweise vor allem bei diskontinuierlichem Betrieb von Vorteil ist.

Demgegenüber kann es bei kontinuierlichem Betrieb vorteilhaft sein, die alternative Fahrweise anzuwenden, bei der Phthalsäureanhydrid in den Reaktor dosiert wird, das durch das im Reaktor entstehende Phthalid verdünnt wird.

Die katalytische Hydrierung wird im allgemeinen bei Temperaturen von 50 bis 400°C, vorzugsweise 100 bis 250°C, insbesondere 140 bis 220°C und Drücken von 1 bis 400 bar, vorzugsweise 5 bis 300 bar, insbesondere 5 bis 200 bar, besonders vorteilhaft 30 bis 120 bar durchgeführt.

Als Reaktoren können übliche Hydrierreaktoren, z.B. Autoklaven oder Rohrreaktoren, verwendet werden.

Als Hydrierkatalysatoren eignen sich beispielsweise Oxide bzw. Metalle der ersten bis vierten Hauptgruppe des Periodensystems der Elemente wie Lithium, Natrium, Kalium, Calcium, Bor, Aluminium, Silicium und Zinn, der ersten bis achten Nebengruppe des Periodensystems der Elemente wie Titan, Zirkon, Vanadin, Niob, Chrom, Molybdän, Mangan, Rhenium, Cobalt, Nickel, Ruthenium, Rhodium, Palladium, Osmium, Iridium, Platin, Kupfer und Silber und der Lanthanidengruppe wie Lanthan, Praseodym, Samarium und Ytterbium oder deren Gemische.

Bevorzugt werden Nickel-Katalysatoren, insbesondere Raney-Nickel, verwendet. Die Hydrierkatalysatoren können z.B. als Trägerkatalysatoren, homogene Katalysatoren oder suspendierte Katalysatoren eingesetzt werden.

Das Gewichtsverhältnis von dem zu hydrierenden Phthalsäureanhydrid zu dem als Lösungsmittel verwendeten Phthalid beträgt im allgemeinen 1000:1 bis 1:1000, vorzugsweise 500:1 bis 1:500, insbesondere 200:1 bis 1:200. Diese Bereiche gelten für die kontinuierliche Arbeitsweise, nachdem sich nach dem Anfahren die für den kontinuierlichen Betrieb geltenden stationären Betriebsbedingungen eingestellt haben.

In einer bevorzugten Ausführungsform des erfindungsgemäßen Verfahrens wird man die erfindungsgemäße Umsetzung abbrechen, sobald alles eingesetzte Phthalsäureanhydrid hydriert ist. Der Zeitpunkt für den Abbruch wird z.B. durch Bestimmung des noch vorhandenen Phthalsäureanhydrids, beispielsweise durch Gas-Chromatographie oder aus dem zeitlichen Verlauf der Wasserstoffaufnahme ermittelt. Bei dieser Arbeitsweise werden Selektivitäten für das gebildete Phthalid von mehr als 80 % erreicht.

Die Aufarbeitung des Reaktionsproduktes erfolgt nach üblichen Methoden, vorzugsweise durch Destillation.

In den Verbindungen I und II bedeuten R¹, R², R³ und R⁴ unabhängig voneinander Wasserstoff; C₁-C₄-Alkyl wie Methyl, Ethyl, n-Propyl, iso-Propyl, n-Butyl, iso-Butyl, sec.-Butyl, tert.-Butyl, bevorzugt Methyl und Ethyl, insbesondere Methyl; C₁-C₄-Alkoxy wie Methoxy, Ethoxy, n-Propoxy, iso-Propoxy, n-Butoxy, iso-Butoxy, sec.-Butoxy, tert.-Butoxy, bevorzugt Methoxy und Ethoxy, insbesondere Methoxy. Besonders bevorzugt bedeuten alle Substituenten R¹, R², R³ und R⁴ Wasserstoff.

Die als Ausgangsstoffe verwendeten Phthalsäureanhydride II sind allgemein bekannt, wie das Phthalsäureanhydrid, oder können nach bekannten Verfahren erhalten werden (J. of Org. Chemistry 51, 3439-3446 (1986); Synthesis 223-224 (1985)).

Die Phthalide I eignen sich z.B. als Ausgangsstoff für die Synthese von Pflanzenschutzmitteln.

Die Erfindung wird durch folgende Beispiele verdeutlicht.

### Beispiel 1

In einem 2-1-Autoklav mit Hubrührer werden 413 g Phthalsäureanhydrid (PSA), 964 g Phthalid (für Versuch 1d 688,5 g PSA und 688,5 g Phthalid) und die in der Tabelle angegebene Menge des Hydrierkatalysators Raney-Nickel als Schmelze vorgelegt. Nach Inertisieren mit Stickstoff und Austausch gegen Wasserstoff wird bei einer Hubzahl von 160 je Minute auf die angegebene Temperatur aufgeheizt und der Wasserstoff auf den angegebenen Druck aufgepreßt. Es wird unter diesen Bedingungen bis zum PSA-Umsatz von größer als 99 % hydriert (Kontrolle durch Gas-Chromatographie).

Die Versuchsbedingungen und Ergebnisse sind in der folgenden Tabelle zusammengefaßt:

| Versuchs-Nr. | Temperatur °C | Druck bar | Katalysatormenge g | Wasserstoffaufnahme Nl | Hydrierzeit min | Umsatz % | Selektivität % |
|---|---|---|---|---|---|---|---|
| 1a | 170 | 40 | 4,13 | 136 | 365 | 100 | 75 |
| 1b | 183 | 80 | 1,03 | 137 | 1162 | 100 | 80 |
| 1c | 183 | 80 | 1,45 | 137 | 435 | 100 | 85 |
| 1d | 183 | 80 | 5,16 | 208 | 164 | 99 | 87 |
| 1e | 180 | 80 | 42,4 | 120 | 80 | 100 | 80,5 |

### Beispiel 2

In einem 0,5-1-Rührautoklav mit Begasungsrührer werden 88,8 g Phthalsäureanhydrid (PSA),207,2 g Phthalid und 0,90 g Raney-Nickel (B 113 N von Degussa) als Schmelze bei ca. 120°C vorgelegt. Nach Einschalten des Rührers wird Wasserstoff auf einen Druck von 40 bar aufgepreßt und auf 180°C erwärmt. Es wird bei diesem Druck und dieser Temperatur bis zum PSA-Umsatz von größer als 99 % hydriert, wobei die Rührdrehzahl 500 (Versuch 2a) bzw. 1000 (Versuch 2b) Umdrehungen pro Minute (Upm) beträgt.

| Versuchs-Nr. | Drehzahl Upm | Hydrierzeit min | Umsatz % | Selektivität % | Wasserstoffaufnahme Nl |
|---|---|---|---|---|---|
| 2a | 500 | 235 | 99,9 | 85 | 25,1 |
| 2b | 1000 | 225 | 99,7 | 83 | 24,8 |

### Zusammensetzung der Produkte in Gew.-%

| Versuchs-Nr. | Phthalid | Wasser | PSA | o-Tolylsäure |
|---|---|---|---|---|
| 2a | 92,3 | 3,3 | <0,1 | 3,2 |
| 2b | 91,9 | 3,4 | 0,1 | 3,7 |

### Beispiel 3

In einem 2-1-Autoklav mit Hubrührer werden 592 g Phthalsäureanhydrid (PSA), 785 g Phthalid und 35 g Raney-Nickel als Schmelze vorgelegt. Nach Inertisieren mit Stickstoff und Austausch gegen Wasserstoff wird bei einer Hubzahl von 160 je Minute auf die angegebene Temperatur aufgeheizt und Wasserstoff auf einen Druck von 80 bar aufgepreßt. Es wird unter diesen Bedingungen bis zum PSA-Umsatz von größer als 99 % hydriert (Kontrolle durch Gas-Chromatographie).

Die Versuchsbedingungen und Ergebnisse sind in der folgenden Tabelle zusammengefaßt:

| Versuchs-Nr. | Temperatur °C | Wasserstoffaufnahme Nl | Hydrierzeit min | Umsatz % | Selektivität % |
|---|---|---|---|---|---|
| 3a | 140 | 172 | 200 | 99 | 82 |
| 3b | 160 | 170 | 160 | 99,5 | 83 |

### Beispiel 4

In der in Beispiel 3 beschriebenen Apparatur wird eine Lösung von 43 Gew.-% Phthalsäureanhydrid (PSA) in Phthalid und wasserfeuchtes Raney-Nickel in einer Menge von 5,9 Gew.-%, bezogen auf PSA, vorgelegt und wie in Beispiel 3 bei einem Druck von 80 bar und einer Temperatur von 140°C hydriert, bis 75 % der theoretisch benötigten Menge an Wasserstoff verbraucht sind. Die Zeit bis zu dieser Wasserstoffaufnahme wird bestimmt.

| Versuchs-Nr. | Zeit bis 75 % Wasserstoffaufnahme min |
|---|---|
| 4a | 117 |
| 4b | 105 |
| 4c | 108 |

### Vergleichsbeispiel (nicht erfindungsgemäß)

Die Versuche gemäß Beispiel 4 werden in analoger Weise wiederholt, wobei anstelle von Phthalid Butyrolacton als Lösungsmittel verwendet wird. Dabei ermittelt man folgende Hydrierzeit.

| Versuchs-Nr. | Zeit bis 75 % Wasserstoffaufnahme min |
|---|---|
| 5a | 285 |
| 5b | 390 |
| 5c | 357 |
| 5d | 380 |

Bei der Verwendung von Butyrolacton als Lösungsmittel für die PSA-Hydrierung ist im Vergleich zur Verwendung von Phthalid als Lösungsmittel (Beispiel 4) eine um den Faktor 2,4 bis 3,5 verlängerte Reaktionszeit erforderlich, um einen vergleichbaren Umsatz zu erzielen.

## Patentansprüche

1. Verfahren zur Herstellung von Phthaliden der allgemeinen Formel I, 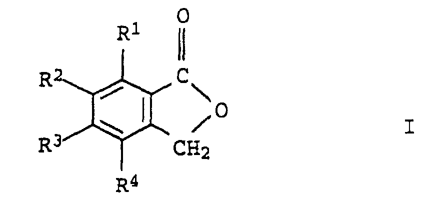 in der R¹, R², R³ und R⁴ unabhängig voneinander Wasserstoff, C₁-C₄-Alkyl oder C₁-C₄-Alkoxy bedeuten, durch katalytische Hydrierung von Phthalsäureanhydriden der allgemeinen Formel II, 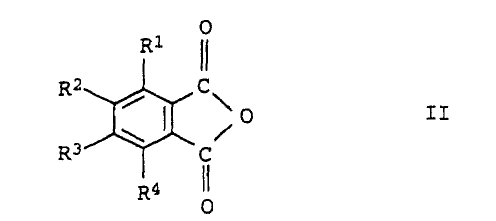 in der die Reste R¹, R², R³ und R⁴ die oben genannten Bedeutungen haben, in einem Lösungsmittel, **dadurch gekennzeichnet, daß** man das Phthalid I, das bei der katalytischen Hydrierung als Reaktionsprodukt entsteht, als Lösungsmittel verwendet.

## Claims

1. A process for preparing a phthalide of the general formula I 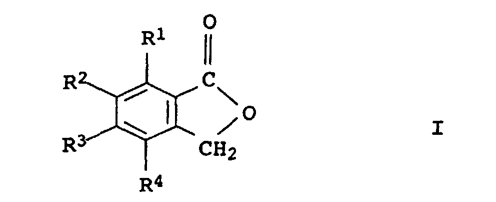 where R¹, R², R³ and R⁴ are each independently of the others hydrogen, C₁-C₄-alkyl or C₁-C₄-alkoxy, by catalytic hydrogenation of a phthalic anhydride of the general formula II 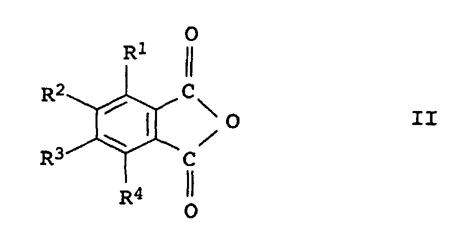 where R¹, R², R³ and R⁴ are each as defined above, in a solvent, which comprises using said phthalide I, the reaction product of the catalytic hydrogenation, as solvent.

## Revendications

1. Procédé pour la préparation de phtalides de formule générale I 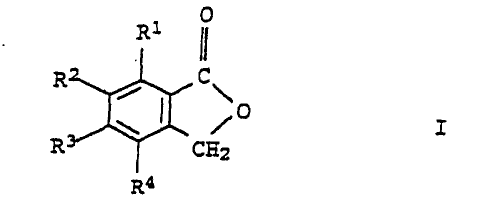 dans laquelle R¹, R², R³ et R⁴ représentent chacun, indépendamment les uns des autres, l'hydrogène, un groupe alkyle en C1-C4 ou alcoxy en C1-C4, par hydrogénation catalytique des anhydrides phtaliques de formule générale II 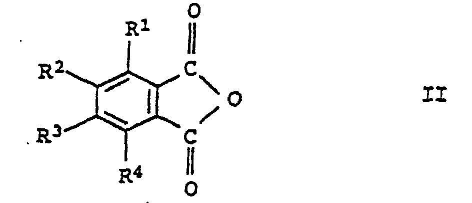 dans laquelle R¹, R², R³ et R⁴ ont les significations indiquées ci-dessus, dans un solvant, ce procédé se caractérisant par le fait que l'on utilise en tant que solvant le phtalide I formé en produit de réaction à l'hydrogénation catalytique.
